# EUROPÄISCHE PATENTANMELDUNG

(11) **EP 0 547 508 A1**
(43) Veröffentlichungstag der Anmeldung: **23.06.1993**
(21) Anmeldenummer: 92121076.1
(22) Anmeldetag: 10.12.1992
(51) Int. Cl.: A61K 31/52

(54) **Verwendung von Xanthinderivaten zur Stabilisierung der Gehirndurchblutungsautoregulation**

(30) Priorität: 11.12.1991 DE 4140805
(71) Anmelder: HOECHST AKTIENGESELLSCHAFT, D-65926 Frankfurt am Main (DE)
(72) Erfinder: Kolvenbach, Ralf, Dr., W-4000 Düsseldorf (DE); Grome, John J., Dr., W-6200 Wiesbaden (DE); Schneider, Ernst-Jürgen, Dr., W-6277 Bad Camberg (DE)

(57) **Zusammenfassung**

Die Verwendung von mindestens einem Xanthinderivat der Formel I, 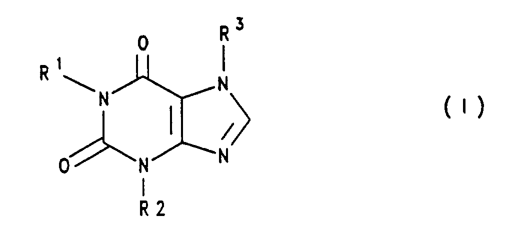
und/oder von mindestens einem physiologisch verträglichen Salz der Formel I, wobei R¹ für Oxoalkyl, Hydroxyalkyl oder Alkyl, R² für Wasserstoffatom oder Alkyl und R³ für Wasserstoffatom, Alkyl, Oxoalkyl oder einem Alkyl mit bis zu 6 Kohlenstoffatomen, dessen Kohlenstoffkette von einem Sauerstoffatom unterbrochen ist, steht, zur Herstellung von Arzneimitteln zur Stabilisierung der Gehirndurchblutungsautoregulation, sowie der Gehirnprotektion bei kardio- oder pulmonaler Reanimation, wird beschrieben.

## Beschreibung

Eine Reihe Von Oxoalkyl- und Hydroxyalkyl-xanthinen wirkten durchblutungsfördernd und kann auch bei cerebralen Durchblutungsstörungen eingesetzt werden (US 4,289,776, PCT 86/00401, US 3,737,433). So ist bekannt, daß 1-(5-Oxohexyl)-3-methyl-7-n-propyl-xanthin (Verbindung 1) aufgrund seiner gefäßerweiternden Wirkung bei geringer Toxizität zur Behandlung von Patienten geeignet ist, die an arteriellen Durchblutungsstörungen leiden. Dort werden ebenfalls Verfahren zur Herstellung dieser Verbindung beschrieben (US 4,289,776).

In der US Patentschrift Nr. 4,719,212 wird die Verwendung von 1-(5-Oxohexyl)-3-methyl-7-n-propyl-xanthin zur Behandlung von Gedächtnisstörungen beschrieben.

Säugetiere sind in der Lage, die Gehirndurchblutung durch Autoregulation über weite Strecken der lokalen Blutdruckskala konstant zu halten. Die Größe des Blutflusses durch das Gehirn über die Aa. carotides und Aa. vertebrales beträgt etwa 750 ml/min. Zu schweren Schäden im Gehirn kann es kommen, wenn die Gehirndurchblutung abfällt, beim gesunden Menschen beispielsweise beim. Unterschreiten des kritischen arteriellen Mitteldrucks von 9,3 KPa (etwa 70 mm Hg) oder beim Überschreiten eines mittleren arteriellen Drucks von 170 mm Hg.

Bei der Durchführung von operativen und diagnostischen Eingriffen wird das Zentralnervensystem reversibel gelähmt, um eine Schmerzfreiheit des Körpers zu erreichen. Mit zunehmender Tiefe der Narkose kann es zu ansteigenden Atem- und Kreislaufdepressionen kommen. Dabei können sich Sauerstoff-Mangelschädigungen ergeben, die mit zunehmenden Funktionsstörungen, reversiblen Funktionsausfall oder irreversiblen Funktionsausfall verbunden sind.

Es wurde nun gefunden, daß die erfindungsgemäßen Xanthinderivate und ihre physiologisch verträglichen Salze geeignet sind, die Autoregulation der Gehirndurchblutungen in einem größeren Bereich zu gewährleisten. Selbst bei hohen Blutdruckspitzen kommt es nicht zum Durchbruch der Bluthirnschranke, und auch bei niedrigen Arteriendruck wird noch eine ausreichende Hirndurchblutung gewährleistet. Es wird also die Autoregulation der Bluthirnschranke stabilisiert, bzw. die Folgen auftretender Störungen in der Autoregulation werden reduziert.

Die Erfindung betrifft daher die Verwendung von mindestens einem Xanthinderivat der Formel I 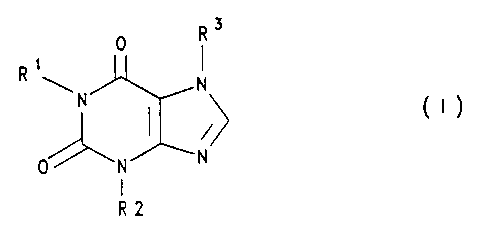
und/oder mindestens einem physiologisch verträglichen Salz der Formel I, wobei
- R¹: für
a) Oxoalkyl mit 3 bis 8 Kohlenstoffatomen, deren Kohlenstoffkette geradkettig oder verzweigt sein kann,
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt, oder
c) Alkyl mit 1 bis 6 Kohlenstoffatomen, deren Kohlenstoffkette geradkettig oder verzweigt sein kann, steht,
- R²: für
a) Wasserstoffatom oder
b) Alkyl mit 1 bis 4 Kohlenstoffatomen, deren Kohlenstoffkette geradkettig oder verzweigt sein kann, steht,
- R³: für
a) Wasserstoffatom,
b) Alkyl mit 1 bis 6 Kohlenstoffatomen, deren Kohlenstoffkette geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann,
steht, zur Herstellung von Arzneimitteln zur Stabilisierung der Gehirndurchblutungsautoregulation.

Bevorzugt werden Xanthinderivate der Formel I und deren physiologisch verträglichen Salze verwendet, wobei
- R¹: für
a) Oxoalkyl mit 4 bis 6 Kohlenstoffatomen, deren Kohlenstoffkette geradkettig ist, oder
b) Alkyl mit 3 bis 6 Kohlenstoffatomen, steht,
- R²: für Alkyl mit 1 bis 4 Kohlenstoffatomen steht,
- R³: für
a) Alkyl mit 1 bis 4 Kohlenstoffatomen oder
b) Oxoalkyl mit 3 bis 6 Kohlenstoffatomen, steht.

Besonders bevorzugt wird 1-(5-Oxohexyl)-3-methyl-7-n-propyl-xanthin eingesetzt. Beispielhaft genannt seien die folgenden Verbindungen der Formel I:
1-(5-Hydroxy-5-methyl-hexyl)-3-methyl-xanthin,
7-(Ethoxymethyl-1-(5-hydroxy-5-methyl-hexyl)-3-methyl-xanthin,
1-(5-Oxohexyl)-3,7-dimethylxanthin,
7-(2-Oxopropyl)-1,3-di-n-butyl-xanthin oder
1-Hexyl-3,7-dimethylxanthin.

Geeignete physiologisch verträgliche Salze der Xanthinderivate der Formel I sind beispielsweise Alkali-, Erdalkali- oder Ammoniumsalze, einschließlich solcher von physiologisch verträglichen organischen Ammoniumbasen.

Die Xanthinderivate der Formel I können nach den folgenden Verfahren hergestellt werden:
a) Umsetzung von Alkalimetallsalzen von 3-Monoalkyl- oder 1,3- oder 3,7-Dialkylxanthinen mit einer Verbindung der Formel II, 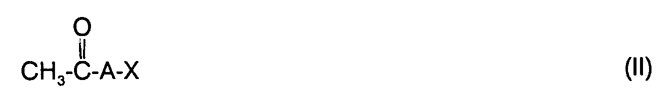 wobei A für eine Alkylgruppe mit 1 bis 6 Kohlenstoffatomen und X für Halogen wie Fluor, Chlor, Brom oder Jod steht, unter basischen Bedingungen,
b) Umsetzung eines 3-Monoalkyl- oder 3,7-Dialkylxanthins mit einer Verbindung der Formel III, 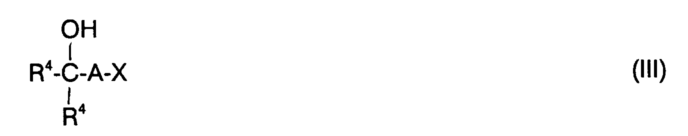 wobei X und A die unter a) genannte Bedeutung haben und R⁴ für Wasserstoff und/oder Methyl steht, unter basischen Bedingungen
c) Umsetzungen der nach a) oder b) erhältlichen Verbindungen, worin R³ für Wasserstoffatom steht mit einer Verbindung der Formel IV,

   CH₃-CₙH₂-O-CₘH₂-X (IV)

   worin n für eine ganze Zahl von O bis 4 und m für eine ganze Zahl von 1 bis 5 steht, unter der Bedingung daß n und m zusammen nicht mehr als 5 sind, und X wie unter a) definiert ist, unter basischen Bedingungen, oder
d) Umsetzung eines Xanthins mit einem entsprechenden Alkylhalogenid in einem Lösungsmittel.

Die obengenannten Umsetzungen erfolgen unter Standardbedingungen auf bekannte Weise (US 4,289,776, PCT/EP86/00401, US 3,737,433, DE 4 217 639).

Die Ausgangsstoffe der Umsetzungen sind bekannt oder lassen sich nach literaturbekannten Methoden leicht herstellen.

Die Erfindung betrifft auch Arzneimittel, die aus mindestens einem Xanthinderivat der Formel I und/oder mindestens einem von deren physiologisch verträglichen Salzen bestehen, neben pharmazeutisch geeigneten und physiologisch verträglichen Trägerstoffen, Verdünnungsmitteln und/oder anderen Wirk- und Hilfsstoffen.

Die erfindungsgemäßen Arzneimittel können oral, topisch, rektal, intravenös oder gegebenenfalls auch parenteral appliziert werden. Die Applikation erfolgt vor, während oder nach einer Operation oder einem überhöhten oder erniedrigten Blutdruck.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwa steriles Wasser und ein- oder mehrwertige Alkohole, z.B. Glycerin, genannt.

Aufgrund der pharmalogischen Eigenschaften der erfindungsgemäßen Xanthinderivate können diese Verbindungen bei allen Operationen in der Klinik oder ambulanten Versorgung angewendet werden, in denen es zu stark erhöhten oder erniedrigten Blutdruckwerten kommen kann, beispielsweise Gefäß- oder Nervenchirugie. Ferner eignen sie sich zur Hirnprotektion bei Kardio- oder pulmonalen Reanimationen.

Vorzugsweise werden die pharmazeutischen Präparate in Dosierungseinheiten hergestellt und verabreicht, wobei jede Einheit als aktiven Bestandteil eine bestimmte Dosis von mindestens einem der Xanthinderivate der Formel I und/oder mindestens einem von deren physiologisch verträglichen Salzen enthält. Bei festen Dosierungseinheiten, wie Tabletten, Kapseln, Dragees oder Suppositorien, kann diese Dosis bis zu etwa 300 mg, bevorzugt jedoch etwa 10 bis 200 mg, betragen.

Für die Behandlung eines Patienten (70 kg), der einen stark überhöhten oder erniedrigten Blutdruck erlitten hat, ist in frühen Phasen eine intravenöse Infusionsbehandlung von maximal 1200 mg pro Tag und in der späteren Rehabilitationsphase eine orale Verabreichung von 3 mal 300 mg pro Tag der Verbindung 1 und/oder der entsprechenden Salze der Verbindung 1 indiziert.

Unter Umständen können jedoch auch höhere oder niedrigere Dosen angebracht sein. Die Verabreichung der Dosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Xanthinderivate der Formel I und/oder deren entsprechende Salze bei der Herstellung der vorgenannten galenischen Zubereitungsformen auch zusammen mit anderen geeigneten Wirkstoffen, beispielsweise Wirkstoffen, die freie Sauerstoffradikale abfangen, z.B. 1,5-Dihydro-4H-Pyrazolo(3,4-d)pyrimidin-4-on oder dem Enzym Superoxiddismutase, formuliert werden.

### Beispiel 1

### Herstellung von 1-(5-Oxohexyl)-3-methyl-7-n-propyl-xanthin (Verbindung 1)

437.2 g 3-Methyl-7-propylxanthin, in einem Gemisch aus 240 g Methanol und 321 g Wasser suspendiert, werden bei erhöhter Temperatur mit 160 g 50%iger Natronlauge in Lösung gebracht, anschließend bei Siedetemperatur mit 358 g 1-Brom-hexanon-(5) versetzt und 4 1/2 Stunden am Rückfluß erhitzt. Nach dem Abkühlen wird nicht umgesetztes 3-Methyl-7-propylxanthin abgetrennt und der Alkohol abdestilliert. Die wässrige Lösung wird mit Natronlauge auf pH 11 gestellt und mit Methylenchlorid extrahiert. Aus dem Rückstand der Methylenchloridlösung wird nach dem Umkristallisieren aus 5.2 l Diisopropylether 1-(5-Oxohexyl)-3-methyl-7-propylxanthin vom Schmelzpunkt 69 - 70° C in etwa 90%iger Ausbeute (bezogen auf umgesetztes 3-Methyl-7-propylxanthin) erhalten.

### Beispiel 2

Es werden 36 Ratten unterteilt in drei Gruppen operiert. Die erste Gruppe dient als Kontrolle, d.h. es wird eine akute Hypertonie erzeugt, ohne daß die Carotiden abgeklemmt werden. Die zweite Gruppe wird während der Ischämie und die dritte Gruppe während der Reperfusion mit Adrenalin stimuliert. In jeder Gruppe werden jeweils 6 Tiere mit der Verbindung 1 als Dauerinfusion behandelt.

Bei einem mittleren arteriellen Druck von 170 mmHg wird in der Kontrollgruppe die cerebrale Autoregulation durchbrochen (entsprechend 3 µg/kg Adrenalin (Firma Sigma)). Die dreifache Dosis führt bei einem Blutdruck von 200 mmHg zum Tod der Tiere. Im Gegensatz dazu ist in der mit Verbindung 1 behandelten Gruppe die dreifache Konzentration und ein mittlerer Druck von 180 mmHg erforderlich um an die Grenze der Autoregulation zu kommen. Der Verschluß beider Carotiden führt bei den Tieren, die mit Verbindung 1 behandelt wurden, zu einer höheren Restdurchblutung (25 %) als bei den Kontrolltieren. Während der Reperfusion bleibt die Gehirndurchblutung in der Kontrollgruppe auf dem niedrigen Niveau von 23 %. Die Autoregulation wird bereits bei 150 mmHg durchbrochen (entsprechend 3 µg/kg/Adrenalin). Die Tiere, die mit Verbindung 1 behandelt wurden, erreichen sofort wieder das präischämische Ausgangsniveau. Erst bei 180 mmHg kommt es zum Durchbruch der Autoregulation.

Alle Kontrolltiere verstarben nach einer Dosis von 10 µg/kg Adrenalin. Die letale Dosis für mit Verbindung 1 behandelte Tiere liegt bei 30 µg/kg Adrenalin. Zusammenfassend läßt sich feststellen, daß die mit Verbindung 1 behandelten Tiere - im Vergleich mit den Kontrolltieren - höhere Blutdruckspitzen und Blutdruckabsenkungen tolerieren und deren Gehirndurchblutungsautoregulation erst bei der 3-fachen Adrenalinkonzentration durchbrochen wird.

## Patentansprüche

1. Verwendung von mindestens einem Xanthinderivat der Formel I, 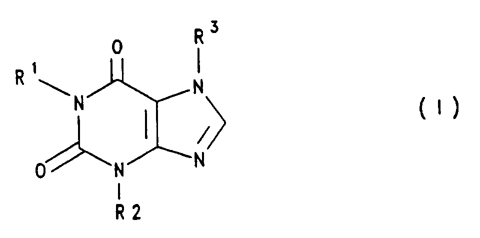 und/oder mindestens einem physiologisch verträglichen Salz der Formel I, wobei
R¹ für
a) Oxoalkyl mit 3 bis 8 Kohlenstoffatomen, deren Kohlenstoffkette geradkettig oder verzweigt sein kann,
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt, oder
c) Alkyl mit 1 bis 6 Kohlenstoffatomen, deren Kohlenstoffkette geradkettig oder verzweigt sein kann, steht,
R² für
a) Wasserstoffatom oder
b) Alkyl mit 1 bis 4 Kohlenstoffatomen, deren Kohlenstoffkette geradkettig oder verzweigt sein kann, steht,
R³ für
a) Wasserstoffatom,
b) Alkyl mit 1 bis 6 Kohlenstoffatomen, deren Kohlenstoffkette geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann,
steht, zur Herstellung von Arzneimitteln zur Stabilisierung der Gehirndurchblutungsautoregulation.

2. Verwendung gemäß Anspruch 1, dadurch gekennzeichnet, daß mindestens ein Xanthinderivat der Formel I, wobei
R¹ für
a) Oxoalkyl mit 4 bis 6 Kohlenstoffatomen, deren Kohlenstoffkette geradkettig ist, oder
b) Alkyl mit 3 bis 6 Kohlenstoffatomen steht,
R² für Alkyl mit 1 bis 4 Kohlenstoffatomen steht,
R³ für
a) Alkyl mit 1 bis 4 Kohlenstoffatomen oder
b) Oxoalkyl mit 3 bis 6 Kohlenstoffatomen, steht und/oder mindestens einem physiologisch verträglichen Salz der Formel I, eingesetzt wird.

3. Verwendung von 1-(5-Oxohexyl)-3-methyl-7-n-propyl-xanthin oder mindestens einem von seinen physiologisch verträglichen Salzen zur Herstellung von Arzneimitteln zur Stabilisierung der Gehirndurchblutungsautoregulation.

4. Verwendung von mindestens einem Xanthinderivat der Formel I gemäß einem oder mehreren der Ansprüche 1 bis 3 zur Herstellung eines Arzneimittels zur Gehirnprotektion bei kardio- oder pulmonaler Reanimation.

5. Arzneimittel zur Stabilisierung der Gehirndurchblutungsautoregulation, gekennzeichnet durch einen wirksamen Gehalt an mindestens einem Xanthinderivat der Formel I 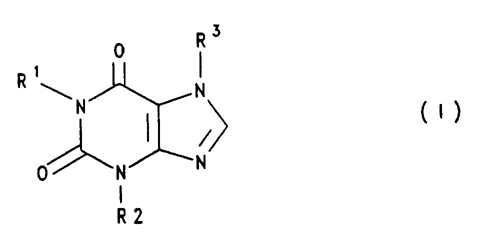 und/oder mindestens einem physiologisch verträglichen Salz der Formel I, wobei
R¹ für
a) Oxoalkyl mit 3 bis 8 Kohlenstoffatomen, deren Kohlenstoffkette geradkettig oder verzweigt sein kann,
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt, oder
c) Alkyl mit 1 bis 6 Kohlenstoffatomen, deren Kohlenstoffkette geradkettig oder verzweigt sein kann, steht
R² für
a) Wasserstoffatom oder
b) Alkyl mit 1 bis 4 Kohlenstoffatomen, deren Kohlenstoffkette geradkettig oder verzweigt sein kann, steht,
R³ für
a) Wasserstoffatom,
b) Alkyl mit 1 bis 6 Kohlenstoffatomen, deren Kohlenstoffkeffe geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann,
steht.

6. Arzneimittel gemäß Anspruch 5, dadurch gekennzeichnet, daß es mindestens ein Xanthinderivat der Formel I, wobei
R¹ für
a) Oxoalkyl mit 4 bis 6 Kohlenstoffatomen, deren Kohlenstoffkette geradkettig ist, oder
b) Alkyl mit 3 bis 6 Kohlenstoffatomen steht,
R² für Alkyl mit 1 bis 4 Kohlenstoffatomen steht,
R³ für
a) Alkyl mit 1 bis 4 Kohlenstoffatomen oder
b) Oxoalkyl mit 3 bis 6 Kohlenstoffatomen steht, und/oder mindestens ein physiologisch verträgliches Salz der Formel I, enthält.

7. Arzneimittel zur Stabilisierung der Gehirndurchblutungsautoregulation, gekennzeichnet durch einen wirksamen Gehalt von 1-(5-Oxohexyl)-3-methyl-7-n-propyl-xanthin oder von mindestens einem seiner physiologisch verträglichen Salze.

8. Arzneimittel gemäß Anspruch 5 zur Gehirnprotektion bei Kardio- oder pulmonaler Reanimation.

9. Verfahren zur Herstellung eines Arzneimittels gemäß einem oder mehreren der Ansprüche 5 bis 8, dadurch gekennzeichnet, daß man mindestens ein Xanthinderivat der Formel I und/oder mindestens einem von deren physiologisch verträgliches Salz der Formel I mit einem physiologisch annehmbaren Träger und weiteren geeigneten Wirk-, Zusatz- oder Hilfsstoffen in eine geeignete Darreichungsform bringt.
